Europäisches Patentamt

European Patent Office

Office européen des brevets

(11) Veröffentlichungsnummer **0 019 822**
**B1**

# EUROPÄISCHE PATENTSCHRIFT

(45) Veröffentlichungstag der Patentschrift:
**27.04.83**

(51) Int. Cl.³: **A 41 D 1/04**, A 61 F 5/00,
A 61 N 1/10

(21) Anmeldenummer: 80102747.5

(22) Anmeldetag: **17.05.80**

(54) **Weste zur therapeutischen Behandlung.**

(30) Priorität: **21.05.79  CH 4739/79**

(43) Veröffentlichungstag der Anmeldung:
**10.12.80 Patentblatt 80/25**

(45) Bekanntmachung des Hinweises auf die Patenterteilung:
**27.04.83 Patentblatt 83/17**

(84) Benannte Vertragsstaaten:
**AT BE DE FR GB IT LU NL SE**

(56) Entgegenhaltungen:
**CA-A-1 053 402**
**CH-A-4 263**
**DE-C-242 782**
**DE-U-7 713 591**
**FR-A-1 065 114**
**FR-A-1 231 117**
**FR-A-1 483 807**
**US-A-3 921 626**

(73) Patentinhaber: **TEMOVA ETABLISSEMENT,
Landstrasse 140, FL-9494-Schaan (LI)**

(72) Erfinder: **Benckhuijsen, Jan Gerrit, Im Pardiel 40,
FL-9494 Schaan (LI)**

(74) Vertreter: **Maas, Friedrich Julius, Dipl.Ing.,
Nidelbadstrasse 96, CH-8803 Rüschlikon (CH)**

Weste zur therapeutischen Behandlung

Die Erfindung betriff eine zur therapeutischen Behandlung bestimmte Weste, aus einem nachgiebigem und der Körperform sich anpassenden elektrostatisch aufladbarem Textilmaterial, mit einer Brust- und Rückenpartie, die längs den Schulter- und Körperseitenlinien zusammenhängen und an deren Unterrand ein elastisches Band angeordnet ist, sowie mit Ärmeln und mit einem längs der Brustpartie und dem Band sich erstreckenden Trennverschluss und mit einer von der Brust- und Rückenpartie ausgehenden, den Hals umfassenden versteiften Halspartie.

Solche Westen weisen eine gesundheitsfördernde Wirkung auf und sind dementsprechend als sogenannte Gesundheitswäsche handelsüblich. Die therapeutische Wirkung beruht hierbei zur Hauptsache auf dem reibungselektrischen Wärmeeffekt, welchen das der Haut anliegende Textilmaterial ausübt. Meist dienen daher diese Westen zur Vermeidung und Linderung etwa von Erkältungskrankheiten, Rheuma, Arthrose und Rückenschmerzen. Eine andere Art von Westen besteht beispielsweise lediglich aus einer Rückenpartie sowie elastischen kurzarmigen Schulterpartien und soll als sogenannte Schultergelenkbandage Beschwerden des Schultergelenks beheben. Erwiesenermassen besitzen diese Westen nur eine beschränkte oder überhaupt keine Wirksamkeit bei der Therapie ernsthafterer Erkrankungen wie degenerativer Veränderungen, Reizzustände und Überlastungsbeschwerden des Schulter-, Armbereichs, z.B. Schulter/Arm-Syndrom, Omarthrose, Schultergelenkarthrose, Schultergelenkkapselentzündung (Periathritis), Humero-Scapularis und anderer bei denen erfahrungsgemäss eine solche äussere Behandlung wenig wirksam ist.

Eine andere bekannte Erkrankung am Oberkörper von Patienten sind Schmerzzustände im Zervikalbereich, etwa ein Zervikalsyndrom. Zwar sind sogenannte Zervikalkragen bzw. Halsstützen bei der Therapie von Verletzungen der Halswirbelsäule und oberen Rückenwirbelsäule gebräuchlich, jedoch beschränkt sich deren Wirksamkeit ausschliesslich auf solche Verletzungen und eine darüber hinausgehende Anwendung von Zervikalkragen als Therapiemittel ist nicht üblich oder ohne praktischen Erfolg.

In den Schweizer Patenten Nr. 514 299 und Nr. 530 179 der Anmelderin ist bereits ein therapeutischer Leibstütz- und Hüftgürtel beschrieben, der insbesondere eine Schmerzlinderung und einen Heileffekt bei Patienten mit Nieren- und Wirbelsäulebeschwerden ausübt. Bekanntlich treten solche Funktionsstörungen in diesen unterschiedlichen Körperregionen oftmals gemeinsam auf. Wie damals überraschend festgestellt wurde, resultiert ein Heileffekt für beide Erkrankungen gleichzeitig bei einem Gürtel, der sowohl einen durch Reibungselektrizität erzeugten Wärmeeffekt als auch eine durch Versteifungen verstärkte Stützwirkung ausübt. Ferner hat sich herausgestellt, dass der Heileffekt dieses Gürtels nur eintritt bei Verwendung eines gestrickten nicht hygroskopischen Textilmaterials aus Polyvinylchlorid- und Acrylfasern mit genügend grosser Maschenweite.

Auf Grund der in grossem Umfange nachgewiesenen Heilerfolge mit diesem therapeutischen Gürtel konnte vermutet werden, dass vielleicht auch die gleichzeitige Behandlung der obengenannten beiden Schmerzzentren im Schulter- und Zervikalbereich eine Steigerung der bisher wenig erfolgreichen äusseren therapeutischen Behandlung ermöglicht.

Die Aufgabe der vorliegenden Erfindung besteht darin, aufgrund der obengenannten Erfahrungen eine therapeutische Heilweste zu schaffen, welche die erwähnten Erkrankungen und Schmerzen des Schulter-, Arm-Bereiches und Zervikalbereiches in wirksamer Weise heilt und lindert.

Diese Aufgabe wird erfindungsgemäss mit einer Weste der eingangs genannten Art gelöst, die dadurch gekennzeichnet ist,

– dass das Textilmaterial ein Gestrick ist, wobei das Textilmaterial aus einem Gemisch langer, auf Kammgarnbasis gesponnener und zur Erzeugung von Reibungselektrizität geeigneter nichthygroskopischer Polyvinylchloridfasern und Acrylfasern besteht,

– die Hals-, Brust- und Rückenpartie je aus einer doppelten Lage des Gestricks besteht, wobei die innere Lage jeweils der Haut anliegt und beide Lagen zueinander bereichsweise verschiebbar sind, und wobei mindestens das Gestrick der Hals- und Rückenpartie eine zum Abdunsten von Hautfeuchtigkeit ausreichende Maschenweite besitzt,

– die Halspartie wenigstens doppelte Halslänge aufweist und derart ausgebildet ist, dass sie nach Umlegung ihres oberen überstehenden Teils auf den unteren Teil eine steife Stütze bildet und gleichzeitig die innere Lage des Gestricks des unteren Teils unter Druck auf der Haut im Hals-, Kiefer- und Hinterhauptbereich anliegt und wobei zwischen dem umgeklappten und unteren Teil ein Zwischenraum für eine Halsstütze gebildet ist.

Die Erfindung wird im folgenden für einige Ausführungsbeispiele anhand der beiliegenden Zeichnung näher erläutert. Es zeigen:

Fig. 1 in vereinfachter Darstellung eine Voransicht einer Weste grösserer Länge,

Fig. 2 in vereinfachter Darstellung eine Rückansicht einer Weste geringerer Länge,

Fig. 3 in schematischer Darstellung eine vergrösserte Ansicht der Halspartie der Weste gemäss Fig. 1, 2 sowie einen Schnitt durch die Halsstütze.

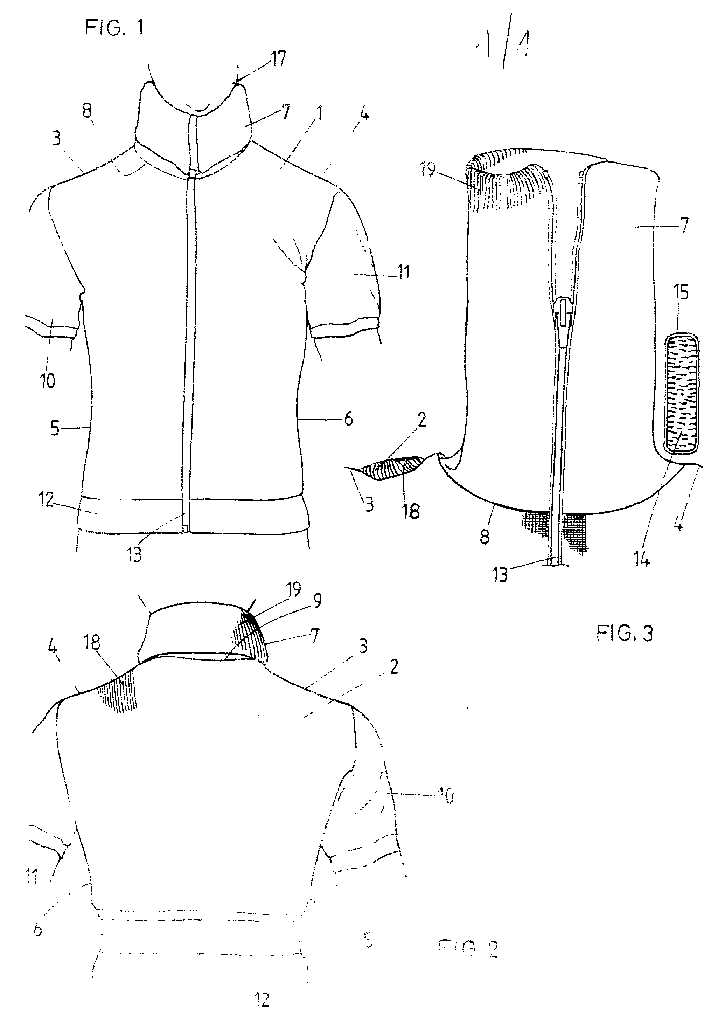

Wie aus Fig. 1 bis 3 ersichtlich ist, bestehen die Westen jeweils aus einer Brust- und Rückenpartie 1 bzw. 2, die längs den Schulterlinien 3, 4 und Körperseitenlinien 5, 6 miteinander vereinigt sind. Da sich die Westen von Fig. 1 und 2 lediglich in ihrer Länge unterscheiden, im übrigen aber hinsichtlich ihrer sonstigen Ausbildung und Beschaffenheit übereinstimmen, werden in Fig. 1 und 2 dieselben Bezugszeichen angewandt. Die Weste verfügt über die in Fig. 3 dargestellte Halspartie 7. Die Halspartie 7 ist an den in der Brust- und Rückenpartie 1, 2 unterhalb des Halsansatzes gerundeten Ausschnitten 8 bzw. 9 angebracht. Im Bereich der Schulterlinien 3, 4 erstrecken sich die Brust- und Rückenpartien 1, 2 bis zur Schulterrundung, von welcher die hier beispielsweise kurzen Ärmel 10, 11 ausgehen. Am Unterrand von Brust- und Rückenpartie 1, 2 befindet sich ein dem Körper anliegendes elastisches Band 12, welches ein Hinaufverrutschen der Weste verhindert. Die vorzugsweise vorderseitig auftrennbare Weste wird von einem Trennverschluss 13, z.B. einem Reissverschluss, zusammengehalten, der das Band 12, die Brustpartie 1 und die Halspartie 7 durchläuft. Dementsprechend umgibt die Weste lückenlos den gesamten Oberkörper einschliesslich der Halsregion.

Ein wesentlicher Bestandteil der Weste bildet deren Halspartie 7 gemäss Fig. 3, wobei die weiter unten noch näher erläuterte Halsstütze 14 nur wahlweise vorzusehen ist. Die Halspartie 7 weist etwa doppelte Länge als der Hals normaler Personen auf und ist so ausgebildet, dass ihr oberer überstehender Teil um den unteren Teil umklappbar ist, wie dies in den Fig. 1 und 2 angedeutet ist. Da das verwendete gestrickte Textilmaterial eine grosse Querelastizität aufweist, übt die umgeklappte Halspartie 7 einen erheblichen Druck auf den Hals des Trägers auf, was zur Erzielung des gewünschten therapeutischen Effektes erwünscht ist. Diese Ausbildung der Halspartie 7 unterscheidet die vorliegende therapeutische Weste von normalen Kleidungsstücken mit sogenannten Rollkragen, bei denen durch die Strickart und die Abmessungen gewährleistet sein muss, dass bei Halsbewegungen nach allen Richtungen nur ein möglichst geringer Widerstand entsteht. Im Gegensatz hierzu soll die umgeklappte Halspartie 7 einen steifen Kragen bilden, der die Kinnpartie abstützt und die Halswirbelsäule vor starken seitlichen Biegungen bewahrt. Zur Erhöhung der Stützwirkung kann in den Zwischenraum, der vom umgeklappten oberen Teil mit dem unteren Teil der Halspartie 7 gebildet wird, eine zusätzliche steife Halsstütze 14 eingelegt werden, die weiter unten noch näher beschrieben wird. Oder es lassen sich Stützelemente, z.B. elastische Verstärkungsstreifen oder Stützstäbchen, zumindest in den unteren Teil der Halspartie 7 einarbeiten. Anders als bei Kleidungsstücken mit üblichen Rollkragen besitzt bei der vorliegenden Weste die Halspartie 7 eine solche Länge, dass der nach dem Umklappen gebildete steife Kragen bis in Höhe der Kieferpartie 17 reicht.

Die zu einem steifen Kragen umgelegte Halspartie 7, ohne oder mit eingelegter Halsstütze 14, ergibt in der beschriebenen Ausbildung zwei wesentliche therapeutische Wirkungen. Der eine Effekt besteht in ihrer Stützfunktion, welche unbedachtsame Kopf- und Körperwendungen in für den Schulter/Arm- und Zervikalbereich schonende Weise abschwächt. Die zweite wesentliche Funktion besteht darin, dass sie als Druckmittel wirkt, welches einen bestimmten andauernden Anlagedruck aufrechthält zwischen der Haut im Halsbereich des Patienten und der ihr anliegenden inneren Lage des Textilmaterials der Halspartie 7. Bekanntlich fördert ein solcher Anlagedruck die Wärmeentwicklung bei einem reibungselektrisch aktiven Textilmaterial, aus welchem die Halspartie 7 besteht. Indem der steife Kragen bis in Kieferhöhe 17 bzw. rückseitig bis unterhalb des Hinterhauptes reicht, sind alle zugehörigen Hautbereiche bis zum Kopfansatz dieser unter Druck erhöhten Wärmeentwicklung ausgesetzt. Infolgedessen herrscht im Hautbereich des Halses und des Nackens einerseits eine konstant erhöhte Temperatur, die beispielsweise durch einen herkömmlichen Pullover mit Rollkragen alleine nicht erzielbar wäre, und andererseits eine heilsame Reizung durch die erzeugten elektrischen Ladungen.

Brust- und Rückenteil 1, 2 sowie die Ärmel 10, 11 der Weste bestehen aus demselben Reibungselektrizität erzeugenden Textilmaterial wie die Halspartie 7. Wie die Erprobung der in Frage kommenden reibungsaktiven Textilmaterialien gezeigt hat, wird der wirksamste Therapieeffekt erzielt mit einem gestrickten Material bestehend aus langen auf Kammgarnbasis gesponnenen Polyvinylchloridfasern, denen ein Anteil von Acrylfasern beigefügt ist. In einer bevorzugten Ausführungsform besteht das Gestrick aus 80 bis 90, vorzugsweise 85% Polyvinylfasern mit dem verbleibenden Rest an Acrylfasern. Beide sind sogenannte Langfasern mit einer Feinheit im Bereich von etwa 3 bis 20 Denier und werden gelegentlich auch als Polyvinylchlorid-Zellwolle bezeichnet. Zu dieser Klasse von Fasern gehören auch Fasern aus nachchloriertem Polyvinylchlorid und aus Vinylchlorid-Azetat-Mischpolymerisaten. Bekanntlich bewirken diese nichthygroskopischen Kunstfasern bei der Bewegung des Textilmaterials gegenüber der Haut eine elektrostatische Aufladung, die ihrerseits ein Wärmegefühl auf den Hautpartien hervorruft, das praktisch unabhängig ist von der Umgebungstemperatur und durch die Bewegungen ständig aufrechterhalten und im Halsbereich in der geschilderten Weise noch erhöht wird.

Der therapeutische Effekt der Weste wird zusätzlich durch die spezielle Struktur des Textilmaterials gesteigert. Wie Fig. 1 und 2 verdeutlichen, schmiegen sich Brust- und Rückenpartie 1, 2 in vollkommenste Weise den Körperformen an. Diese Anpassfähigkeit resultiert aus einer in das Textilmaterial des Rückenteils 2 eingewirkten, in Fig. 2 angedeuteten Längsstruktur 18, die einen elastischen Zug in Querrichtung der Weste ausübt. Gleichermassen ist die Halspartie 7 mit einer Längsstruktur 19 versehen, so dass dieselbe elastisch der Halsregion anliegt. Insbesondere vorteilhaft ist, dass alle Textilpartien der Weste, also Brust- und Körperpartie 1, 2, Ärmel 10, 11 sowie die Halspartie 7 aus einer doppelten Lage des Textilmaterials bestehen. Diese Doppellage ist beispielsweise in Fig. 3 am Oberrand der Halspartie 7 aus dem Verlauf der Längsstruktur 19 erkennbar. Die innere Lage des Textilmaterials liegt direkt auf der Haut auf und erfährt durch die Reibung gegenüber der Haut eine elektrostatische Aufladung. Diese wird verstärkt durch die die äussere Lage des Textilmaterials, die gegen die innere Lage reibt. Das Textilmaterial besitzt eine ausreichende Maschenweite, so dass Feuchtigkeit der Haut, die durch die Wärmewirkung entsteht, durch beide Lagen hindurch abdunsten kann, was nicht nur ein Ankleben des Textilmaterials und ein unangenehmes Gefühl beim Tragen verhindert, sondern insbesondere ein rasches Abfliessen bzw. eine Verminderung der elektrostatischen Ladungen vermeidet. Die durch das doppellagige Textilmaterial erzeugten hohen elektrostatischen Ladungen, die bis zu 30 000 Volt betragen können, bewirken ausser der Erwärmung auch auf und unter der Haut einen Reizeffekt auf entzündete Gewebeteile. Zur Erzeugung hoher Ladungen sollte das Textilmaterial möglichst dicht sein und andererseits die obenerwähnte ausreichende Maschenweite besitzen.

Es wurde ermittelt, dass alle Erfordernisse an die Struktur und Strickart des Textils in den einzelnen Bereichen der Weste am besten erfüllt werden, wenn für die Rückenpartie 2 und Halspartie 7 jeweils ein Feinrippgestrick und für das Brustteil 1 ein Interlockgestrick der nachfolgenden Kenndaten verwendet wird:

Das Feinrippengestrick wurde aus der vorstehend angegebenen Kunstfaser mit 2,5 bis 4 Denier auf einer Rundstrickmaschine bei Verarbeitung mit 10 Nadeln auf 2 cm erzeugt. Die Stichzahl betrug 20 Stiche/2 cm Länge und 14 Stiche/2 cm Breite, die Maschenzahl 70/cm² und das Gewicht 160 g/m². Allgemein wurden als wirksamste Stichzahlbereiche 18–25 bzw. 12–20 Stiche pro 2 cm Länge bzw. Breite und 100–400 g/m² als bevorzugter Gewichtsbereich für das Feinrippgestrick ermittelt. Zu seiner Herstellung kommen beliebige Rundstrickmaschinen oder Flachstrickmaschinen in Frage, da die doppellagigen Partien sowohl in Form eines Schlauches gestrickt und dann eingestülpt oder auch durch Vernähen hergestellt werden können. Bei der als Interlockgestrick herzustellenden Brustpartie 1 wird eine Verarbeitung mit 16 Nadeln auf 2 cm bevorzugt, das Garngewicht beträgt 50 000 m Länge/kg, die Stichzahl 25 bzw. 26 Stiche pro 2 cm Länge bzw. Breite und das Textilgewicht 150–400 g/m².

Wie oben bereits erwähnt, ist es vorteilhaft, den durch Umlegen des oberen überstehenden Teils der Halspartie 7 erzeugten Zwischenraum zum Einlegen einer Halsstütze 14 zu benützen, um die Steifigkeit der Halspartie 7 zu vergrössern. Als Halsstütze 14 dient ein länglicher Streifen aus nachgiebigem, aber formfesten Material, dessen beide Ende mit einem verstellbaren selbstklebenden Haftverschluss bekannter Art versehen sind und eine Anpassung der Halsstütze 14 an unterschiedliche Halsstärken gestattet. An das Material der Halsstütze 14 besteht die Hauptforderung darin, dass es ungehindert Luft passieren lässt. Diese Luftdurchlässigkeit ist notwendig, damit die aus dem doppellagigen unteren Teil der Halspartie 7 austretende abgedunstete Hautfeuchtigkeit die Halsstütze 14 passieren kann. Andernfalls würde im Halsbereich die wegen der Wärmeentwicklung gebildete starke Hautfeuchtigkeit verbleiben und ausser einem Kältegefühl die Bildung von elektrostatischer Ladung wesentlich beeinträchtigen. Diese Forderungen an das Material der Halsstütze 14 werden allgemein mit einem Schaumkörper der Gruppe Polyurethan-Schaum erfüllt, der infolge seiner offenen Poren luftdurchlässig ist und Feuchtigkeit durchlässt, und eine elastische Formfestigkeit aufweist, die einen allseitigen Anlagedruck der Halsstütze 14 gegen den unteren Teil der Halspartie 7 gewährleistet. Der Schaumkörper der Halsstütze 14 besitzt z.B. 1,5–2 cm Dicke und ein Gewicht von 40 kg/m³.

Vorteilhafterweise ist die Halsstütze 14 allseitig mit einer luftdurchlässigen Umhüllung 15 versehen, die vorzugsweise aus einem Gestrick desselben reibungselektrisch wirksamen Textils besteht wie die Halspartie 7. Hierdurch wird die elektrostatische Aufladung mindestens des unteren Teils der Halspartie 7 durch Reibung an der Umhüllung 15 verstärkt. Es können auch mehrere Lagen des Textils als Umhüllung dienen. Ferner kann die Halsstütze eingebaute Versteifungsstäbchen enthalten, falls die Erkrankung, z.B. eine Verletzung der Halswirbelsäure, dies erfordert. Anstelle eine getrennte Halsstütze 14 vorzusehen lässt sich ein Polyurethanschaum als Stütze auch in die Halspartie 7 integrieren.

Die vorliegende therapeutische Weste ersetzt mit der Halsstütze 14 der beschriebenen Art die herkömmlichen Zervikalkragen, die weder porös sind noch aus elektrostatisch aufladbaren Materialien bestehen, also nur eine Stützwirkung besitzen, nicht aber die oben beschriebenen therapeutischen Effekte zeigen. Es ist auch nicht empfehlenswert, solche Zervikalkragen bisher bekannter Bauart im Zwischenraum der Halspartie 7 zu benützen, da dann die Abdunstung von Feuchtigkeit wegen fehlender Porosität dieser Zervikalkragen beeinträchtigt werden kann.

Bei der Erprobung der vorstehenden beschriebenen Weste als Therapiemittel gegen die eingangs genannten Erkrankungen und Schmerzempfindungen wurde eine überraschende Heilwirkung und Schmerzlinderung nachgewiesen. Weil eine derartige Heilwirkung bei den bekannten Westenarten ausgeblieben war, ist es offenkundig, dass die Ursache für die therapeutische Wirksamkeit in der erfindungsgemässen Ausbildung der Weste sowohl im Bereich der Halsregion als auch im Schulterbereich liegt. Offenbar resultiert aus dem Stützeffekt in der Halsregion in Verbindung mit der starken Temperaturerhöhung infolge des Wärmeeffekts und der elektrostatischen Hautreizung ein zusätzlicher unvorhersehbarer Therapieeffekt, der auch eine Fernwirkung in die von der Halsregion abgelegenen Körperregionen des Schultergelenks und des Ellbogengelenks ausübt. Es hängt dies offenbar mit der gegenseitigen nervlichen, vasalen und lympnatischen Beeinflussung des Zervikal- und Schulter-Armbereiches zusammen. Hierbei ist wesentlich, dass weder die Stützwirkung noch den Wärmeeffekt allein den Therapieeffekt hervorrufen, sondern erst deren Kombination bei Anwesenheit hoher elektrostatischer Ladungen.

Nach bisherigen Erfahrungen ist die erfindungsgemässe Weste therapeutisch wirksam bei
- Zervikobrachialsyndrom im weitesten Sinne,
- chronischer, subchronischer und akuter schmerzhafter Schultersteife bzw. weichteilrheumatischen Erkrankungen im Zervikoscapularbereich,
- Zervikalsyndrom mit Ausstrahlungen in die Segmente oder mit akuten Erscheinungen der Nackensteife,
- motorischen Bewegungseinschränkungen durch Schmerz,
- Omarthrose
- Periarthritis humeroscapularis.

Die längere Ausbildung der Weste gemäss Fig. 1 lässt sich gegebenenfalls auch bei Erkrankungen des Wirbelsäulen- und Brustbereiches einsetzen.

Falls erwünscht, kann anstelle eines Reissverschlusses als Trennverschluss 13 auch ein Knopfverschluss vorgesehen sein. Die Weste kann eine der Haut, Wäsche oder Kleidung angepasste Farbgebung aufweisen. An sich sind ferner alle zur Erzeugung von Reibungselektrizität geeignete Kunstfasern zur Herstellung des Textilmaterials verwendbar. Es sei auch noch erwähnt, dass die oben beschriebene Weste, ungeachtet ihrer therapeutischen Zweckbestimmung, für den Träger einen weiteren erheblichen Vorteil besitzt. Während bisher ein Zervikalkragen sowohl weibliche als auch männliche Träger sofort als Kranke erscheinen lässt und dementsprechend eine erhebliche psychologische Belastung im beruflichen und privaten Dasein zur Folge hat, erscheint die beschriebene therapeutische Weste wie ein normales, gut aussehendes Kleidungsstück mit einem sogenannten Rollkragen, was noch verstärkt wird durch die Möglichkeit der Herstellung in verschiedenen modischen Färbungen.

Die Wirksamkeit der erfindungsgemässen Weste ist erprobt worden und aus dem nachstehenden Beispiel ersichtlich, das einem fachärztlichen Gutachten entnommen wurde. Die hierin auftretende Bezeichnung VIBROSTATIC-Schultergelenkbandagen beziehen sich auf die vorstehende Weste und VIBROSTATIC ist ein der Anmelderin geschütztes Warenzeichen.

Beispiel
(Dr. med. D. v. A., Klinik und Institut für Physikalische Therapie Nürnberg)
Die Vibrostatik$^R$-Schultergelenks-Bandagen in Spezialausführung mit angearbeitetem Rollkragen kamen bei uns bei entsprechenden Krankheitsbildern zur Anwendung.

Die Wirkung bei den therapieresistenten und erfahrungsgemäss schwierig zu behandelnden Cervicalsyndromen bei schmerzhafter Schultersteife waren erstaunlich. Gerade diese Patienten klagen häufig über gesteigerte Temperaturempfindlichkeit. Während bei den bisherigen Behandlungen lediglich durch hohe Medikamentengabe oder intra- bzw. periartikuläre Injektionen eine Linderung erreicht werden konnte, wurde jetzt durch die Daueranlage der Vibrostatik$^R$-Schultergelenks-Bandage mit dem Cervicalkragen eine schnelle Besserung der Schmerzen, objektiv eine Besserung der Beweglichkeit im Hals-Wirbelsäule-Schulter-Bereich, in der Schulter, vor allem eine Tonusminderung der hypertonen betroffenen Muskulatur im Segmentbereich der mittleren und unteren Hals-Wirbelsäule-Schulter erreicht. Auch die meist nur diskreten, dafür aber subjektiv so ausserordentlich störenden neurologischen Begleiterscheinungen einer anfänglichen Hyperpathie, Parästhesien bis hin zur objektiv nachweisbarer Kraftminderung, liessen sich eindeutig und gegenüber den bisherigen Verfahren auffallend schnell bessern.

Freilich fanden extendierende Massnahmen, im klinischen Bereich auch als Dauermassnahme, auch stundenlange Extensionen über die Glissonschlinge oder über entsprechende Cervicalkragen Anwendung. Den auffallenden Erfolg mit der Vibrostatik$^R$-Schultergelenksbandage einschliesslich des Vibrostatik$^R$-Cervicalkragens möchte ich hiermit den oben geschilderten elektro-physikalischen Eigenschaften zuschreiben, wobei sicher hier die hohe Wärmeisolation und die Wasser (Schweiss) abstossende Fähigkeit der Faser eine schwer messbare, aber entscheidende Rolle spielt.

**Patentansprüche**

1. Zur therapeutischen Behandlung bestimmte Weste, aus einem nachgiebigen und der Körperform sich anpassenden, elektrostatisch aufladbaren Textilmaterial, mit einer Brust- und Rückenpartie (1, 2), die längs den Schulter- und Körperseitenlinien (3, 4 bzw. 5, 6) zusammenhängen und an deren Unterrand ein elastisches Band (12) angeordnet ist, sowie mit Ärmeln (10, 11), mit einem längs der Brustpartie (1) und dem Band (12) sich erstreckenden Trennverschluss (13), und mit einer von der Brust- und Rückenpartie (1, 2) ausgehenden, den Hals umfassenden versteiften Halspartie (7), dadurch gekennzeichnet,
- dass das Textilmaterial ein Gestrick ist, wobei das Textilmaterial aus einem Gemisch langer, auf Kammgarnbasis gesponnener und zur Erzeugung von Reibungselektrizität geeigneter nichthygroskopischer Polyvinylchloridfasern und Acrylfasern besteht,
- die Hals-, Brust- und Rückenpartie (1, 2, 7) je aus einer doppelten Lage des Gestricks besteht, wobei die innere Lage jeweils der Haut anliegt und beide Lagen zueinander bereichsweise verschiebbar sind, und wobei mindestens das Gestrick der Hals- und Rückenpartie eine zum Abdunsten von Hautfeuchtigkeit ausreichende Maschenweite besitzt,
- die Halspartie (7) wenigstens doppelte Halslänge aufweist und derart ausgebildet ist, dass sie nach Umlegung ihres oberen überstehenden Teils auf den unteren Teil eine steife Stütze bildet und gleichzeitig die innere Lage des Gestricks des unteren Teils unter Druck auf die Haut im Hals-, Kiefer- und Hinterhauptbereich anliegt und wobei zwischen dem umgeklappten und unteren Teil ein Zwischenraum für eine Halsstütze (14) gebildet ist.

2. Weste nach Anspruch 1, gekennzeichnet durch eine zusammenfügbare Halsstütze (14) in Halslänge aus einem formbeständigen, nachgiebigen Materialstreifen aus luftdurchlässigem Polyurethanschaum.

3. Weste nach Anspruch 2, gekennzeichnet durch eine allseitige Umhüllung (15) des Materialstreifens aus einem Gestrick gleicher Art wie die übrigen Teile der Weste.

4. Weste nach einem der Ansprüche 1 bis 3, dadurch gekennzeichnet, dass wenigstens das Gestrick der Rücken- und Halspartie (2, 7) eine Längsstruktur (18, 19) aufweist, welche in Querrichtung einen elastischen Zug ausübt.

5. Weste nach Anspruch 1, gekennzeichnet durch eine Länge der Halspartie (7) derart, dass durch Umklappen des überstehenden oberen Teils ein steifer Kragen entsteht, dessen Oberkante bis dicht an die Kieferpartie reicht.

## Revendications

1. Gilet pour traitement thérapeutique, composé d'un textile souple, s'adaptant à la forme du corps et pouvant se charger électro-statiquement, qui comporte un devant et un dos (1, 2), qui sont réunis le long des lignes des épaules et du corps (3, 4 et 5, 6) sur le bord inférieur desquels est disposé une bande élastique (12) ainsi que des manches (10, 11), une fermeture à glissière (13) s'étendant sur le devant (1) en le séparant en deux parties ainsi que la bande (12) de même qu'un col rigidifié (7) partant du devant et du dos (1, 2), caractérisé par le fait que
– le textile est un tricot, composé d'un mélange de fibres non hygroscopiques de chlorure de polyvinyle et acryliques, ces fibres étant longues, filées à base de peigné et aptes à produire de la tribo-électricité;
– le col, le devant et le dos (1, 2, 7) ont chaque fois deux épaisseurs de tricot, l'épaisseur intérieure étant appliquée chaque fois sur la peau et les deux épaisseurs pouvant être déplacées par zones l'une par rapport à l'autre, tandis qu'au moins le tricot du col et du dos présente une largeur de maille suffisante pour l'évaporation de l'humidité de la peau;
– le col (7) présente une longueur au moins double de celle du cou et il est conçu de manière à former, après le rabattement de sa partie supérieure dépassant sur la partie inférieure un support rigide et de manière à ce qu'en même temps l'épaisseur intérieure du tricot de la partie inférieure soit appliquée, sous pression, sur la peau, dans la région du cou, de la mâchoire et de l'occiput, un espace intermédiaire étant formé entre la partie rabattue et la partie inférieure, pour un support de cou (14).

2. Gilet suivant la revendication 1, caractérisé par un support de cou (14) pouvant être combiné à la longueur du cou et composé d'une bande souple et de forme constante en mousse de polyuréthane perméable à l'air.

3. Gilet suivant la revendication 2, caractérisé par une enveloppe (15) entourant de tous côtés la bande et faite d'un tricot de même nature que les autres parties du gilet.

4. Gilet suivant une des revendications 1 à 3, caractérisé par le fait qu'au moins le tricot du dos et du col (2, 7) présente une structure longitudinale (18, 19) qui exerce transversalement une traction élastique.

5. Gilet suivant la revendication 1, caractérisé par une longueur du col (7) telle que le rabattement de la partie supérieure qui dépasse crée un bourrelet rigide, dont le bord supérieur remonte jusqu'en dessous de la mâchoire.

## Claims

1. Vest for therapeutic treatment adapted to closely fit the body of a wearer and being made of an elastic, electrostatically chargeable textile fabric, having front and back parts (1, 2) joined at the shoulder and side lines (3, 4 and 5, 6) of the trunk, an elastic band (12) at the lower edge, sleeves (10, 11), a separating fastener (13) along the front part (1) and the band (12), and a stiffened collar part (7) extending from the front and back parts (1, 2) and covering the neck, characterized by

– a knitted textile fabric comprising a mixture of long, non-hygroscopic polyvinyl chloride fibres and acrylic fibres spun on a worsted basis and suitable for generating static electricity on frictional movement against the skin,

– said front, back and collar parts (1, 2, 7) each consisting of a double layer of the knitted fabric wherein the inner layer fits close to the skin, both layers are movable in sections in relation to each other, and at least the collar and back parts having a mesh sufficiently large to allow evaporation of skin moisture,

– said collar part (7) being at least twice as high as the neck and designed in such a way as to provide a stiff support for the neck when the top half is folded down onto the lower half while at the same time the inner layer of the textile fabric of the lower half adheres to the skin of the neck, jaw and occipital regions exerting pressure, and wherein a pocket for a neck support (14) is provided between the folded down upper and lower halves.

2. Vest according to claim 1, characterized by a dismountable neck support (14) as long as the neck, and made of a dimensionally stable, resilient strip of material made of polyurethane foam permeable to air.

3. Vest according to claim 2, characterized by a complete cover (15) of the strip of material comprising the same knitted textile fabric as the other parts of the vest.

4. Vest according to one of the claims 1 to 3 wherein at least the knitted textile fabric of the back and collar parts (1,7) have a longitudinal structure which exerts an elastic pull in transverse direction.

5. Vest according to claim 1 wherein the length of the collar part (7) is such that folding down the excess top half creates a stiff collar the top edge of which fits close to the mandibular region.

FIG. 1

1/4

FIG. 3

FIG. 2